# EUROPEAN PATENT APPLICATION

(11) **EP 1 691 312 A2**
(43) Date of publication of application: **16.08.2006**
(21) Application number: 06002926.1
(22) Date of filing: 14.02.2006
(51) Int. Cl.: G06F 19/00

(54) **Surgery data display device, surgery data storing device, and surgery data storing display method**

(30) Priority: 15.02.2005 JP 2005038115
(71) Applicant: Olympus Corporation, Shibuya-ku, Tokyo (JP)
(72) Inventor: Ozaki, Takashi, Hachioji-shi Tokyo 192-8512 (JP); Akinobu, Uchikubo, Hachioji-shi Tokyo 192-8512 (JP); Koichi, Tashiro, Hachioji-shi Tokyo 192-8512 (JP); Chie, Imamiya, Hachioji-shi Tokyo 192-8512 (JP); Takeaki, Nakamura, Hachioji-shi Tokyo 192-8512 (JP); Masakazu, Gotanda, Hachioji-shi Tokyo 192-8512 (JP)
(74) Representative: von Hellfeld, Axel

(57) **Abstract**

A surgery data storing device (101) according to the present invention comprises a surgery data storing section which associates surgery data to a predetermined time and stores this to a storing device, and an event data storing section (S2) which associates the event data to the predetermined time according to an occurrence of a predetermined event and stores this.

## Description

### BACKGROUND OF THE INVENTION

### 1. Field of the Invention

The present invention relates to a surgery data storing device, a surgery data display device, and a surgery data storing display method, and specifically relates to a surgery data display device, a surgery data storing device, and a surgery data storing display method which simplifies the analysis of surgery data.

### 2. Description of the Related Art

Conventionally, surgery data such as living body data at the time of surgery, such as the pulse, blood pressure, and so forth of the patient, and medical equipment data for an electrosurgical knife and so forth which are used for surgery, are stored or recorded. The purpose for storing surgery data is to provide it in the event the status of the patient changes after surgery, for example in the event that an operator needs to confirm surgery content. In such an event, by displaying the various data stored at the time of surgery on a display device and observing this data, the operator can confirm the surgery content at the time of surgery after the surgery has ended.

A technique for storing such data at the time of surgery is proposed in Japanese Unexamined Patent Application Publication No. 2002-233535.

However, even if the operator tries to confirm surgery content after the surgery while displaying such stored surgery data on a display device, the stored surgery data is data only stored chronologically, and therefore each type of data must be viewed following the course of time, and confirming the surgery content can take a long time. Particularly, if the surgery time was long, it takes a long time to analyze the stored surgery data after the surgery, and determining surgery content also takes a long time.

Accordingly, it is an object of the present invention to provide a surgery data storing device by which confirmation of surgery content can be easily performed.

### SUMMARY OF THE INVENTION

A surgery data display device according to the present invention comprises a surgery data storing section for associating surgery data to a predetermined time and storing on a storing device, an event data storing section for storing event data associated with the predetermined time, according to the occurrence of a predetermined event, a surgery data displaying section for displaying surgery data associated with the predetermined time and stored, following a course of time, on a display device, and an event mark displaying section for displaying an event mark, which shows there is stored event data, together with the course of time, on the display device.

A surgery data storing device according to the present invention comprises a surgery data storing section for associating surgery data to a predetermined time and storing to a storing device, and an event data storing section for storing event data associated with the predetermined time, according to the occurrence of a predetermined event.

The surgery data display device according to the present invention comprises a surgery data displaying section for displaying surgery data associated with the predetermined time and stored, following the course of time, on the display device, and an event mark displaying section for displaying an event mark, which shows there is stored event data associated to the predetermined time data according to the occurrence of the predetermined event, and displays this together with the course of time on the display device.

With a method for storing and displaying surgery data according to the present invention, surgery data which includes at least living body data about a patient and output data from medical equipment is associated with predetermined time data and stored in a storing device, event data associated with the predetermined time data is stored according to the occurrence of a predetermined event, the surgery data is displayed on a display device, following the predetermined course of time, and an event mark showing there is stored event data, which is associated to the predetermined time data according to the occurrence of a predetermined event, is displayed on the display device together with the predetermined course of time.

### BRIEF DESCRIPTION OF THE DRAWINGS

Fig. 1 is a configuration diagram showing the configuration of a surgery system according to an embodiment of the present invention;
Fig. 2 is a flowchart of an event data storing process according to an embodiment of the present invention;
Fig. 3 is a diagram showing an example of an event code data table according to an embodiment of the present invention, wherein the correlation between event content and event codes are set;
Fig. 4 is a diagram showing an example of an event data table according to an embodiment of the present invention;
Fig. 5 is a configuration diagram showing the configuration of a surgery data display device according to an embodiment of the present invention;
Fig. 6 is a diagram showing a first display example of one piece of surgery data graphed and displayed;
Fig. 7 is a flowchart showing an example of a process flow wherein the surgery data and event data are graphed and displayed on the screen of the display device;
Fig. 8 is a diagram showing an example of a mark data table for the purpose of setting beforehand a mark to correspond to the event content;
Fig. 9 is a diagram showing a second display example of the surgery data graphed and displayed;
Fig. 10 is a diagram showing a third display example of the surgery data graphed and displayed;
Fig. 11 is a diagram showing a fourth display example of the surgery data graphed and displayed; and
Fig. 12 is a diagram showing a fifth display example of the surgery data graphed and displayed.

### DESCRIPTION OF THE PREFERRED EMBODIMENTS

An embodiment of the present invention will now be described with reference to the drawings.

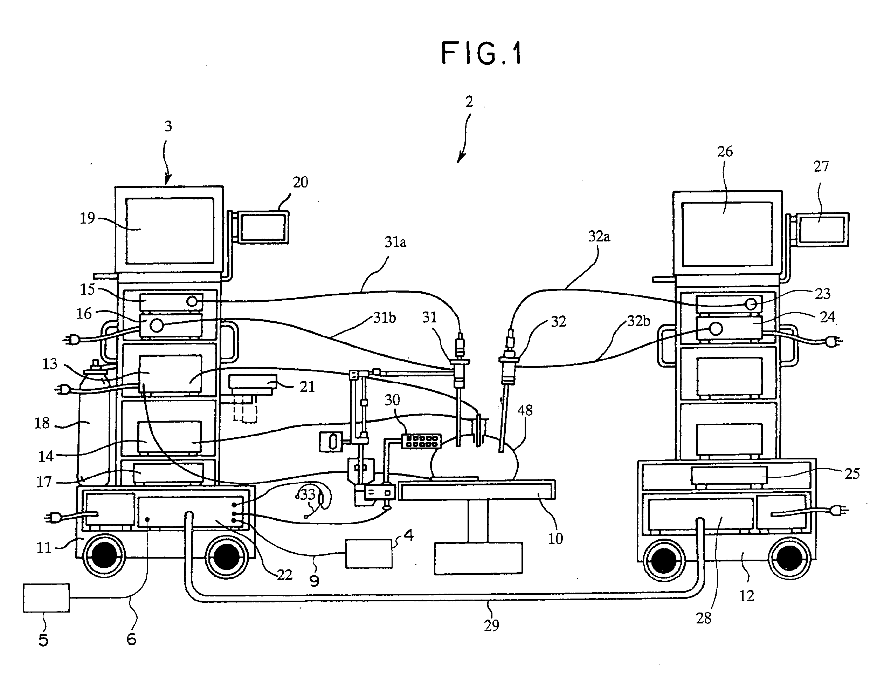

First, an overall configuration of an endoscope surgery system which is a medical system located in an operating room will be described. Fig. 1 is a configuration diagram showing a surgery system according to an embodiment of the present invention.

As illustrated in Fig. 1, a patient bed 10 on which a patient 48 lies down, and an endoscope surgery system 3 are located in an operating room 2. The endoscope surgery system 3 has a first cart 11 and a second cart 12.

The first cart 11 has loaded thereupon medical equipment which are controlled devices, such as an electrosurgical knife device 13, pneumoperitoneum device 14, an endoscope camera device 15, a light source device 16, and a video tape recorder (VTR) 17, and also a gas cylinder 18 filled with carbon dioxide. The endoscope camera device 15 is connected to a first endoscope 31 via a camera cable 31a. The light source 16 is connected to the first endoscope 31 via a light guide cable 31b.

Also, the first cart 11 has loaded thereupon a display device 19, a first intensive display panel 20, and an operation panel 21, and so forth. The display device 19 is for example a TV monitor which displays the endoscope images and the like.

The intensive display panel 20 is a display device which can selectively display various data during the surgery. The operation panel 21 is configured for example with a display unit such as a liquid crystal display and for example an integrated touch panel provided on this display unit, and the nurses in the non-sterilized area operate this central operation device.

Further, a system controller 22 which is a control device is also loaded on the first cart 11. This system controller 22 is connected to the above-described electrosurgical knife device 13, pneumoperitoneum device 14, endoscope camera device 15, light source device 16, and VTR 17 via a communication cable (not shown). The system controller 22 can be connected to a headset-type microphone 33, and the system controller 22 has voice recognition functionality, and can recognize the voice input from the microphone 33, so that the various devices can be controlled by the voice of the operator.

The system controller 22 is a storing device for storing various data, and here has an internal hard disk device (hereafter referred to as "HDD") 22a. As will be described later, the event data, along with the patient living body data and the surgery equipment data for the electrosurgical knife device and the like is stored on the HDD 22a, under the control of the system controller 22a. As also described later, the system controller 22 is a surgery data storing device for associating the surgery data to the elapsed time of the surgery, and storing this on the HDD 22a.

On the other hand, an endoscope camera device 23 which is a controlled device, a light source device 24, an image processing device 25, a display device 26, and a second intensive display panel 27 are placed on the second cart 12.

The endoscope camera device 23 is connected to the second endoscope 32 via a camera cable 32a. The light source device 24 is connected to the second endoscope 32, via a light guide cable 32b.

The display device 26 displays the endoscope images and so forth that are captured on the endoscope camera device 23. The second intensive display panel 27 can selectively display various data during surgery.

The endoscope camera device 23, light source device 24 and image processing device 25 are connected to a relay unit 28 placed on the second cart 12 via a communication wire not shown. Also, the relay unit 28 is connected to a system controller 22 placed on the above-described first cart 11, via a relay cable 29.

Thus, the system controller 22 centrally controls the endoscope camera device 23, light source device 24, and image processing device 25 on the second cart 12, and the electrosurgical knife device 13, the pneumoperitoneum device 14, the endoscope camera device 15, the light source device 16, and the VTR 17 on the first cart 11. Thus, in the case that communication is performed between the system controller 22 and these devices, the system controller 22 can display setting screens such as the setting status of the connected devices or the operation switches and so forth, on the liquid crystal display on the operation panel 21. Further, the system controller 22 can have operation input performed such as changes to the setting values, by desired operation switches of the operation panel 21 being touched and the touch panel of the specified region being operated.

A remote controller 30 is a second central operating device to be operated by surgeons in a sterilized area, and the other devices which are in communication can be operated via the system controller 22.

This system controller 22 is connected to a patient monitor system 4 by a cable 9, and as described below, the physical information acquired from the patient monitor system 4 is stored on the HDD 22a and further analyzed, and the analysis results are displayed on the desired display device 19.

Also, the system controller 22 is connected to a receiving device 5 for a solids recognition / solids identifier device, for example an RFID (Radio Frequency Identification: a contact-free automatic identifier by wireless frequency), via a cable 6, and the system controller 22 can read the data without contact which shows the types of equipment such as endoscopes and treatment instruments from the RFID tags provided on the equipment such as endoscopes and treatment instruments.

Further, an infrared communications port (not shown) which is a communications means is affixed to the system controller 22. The infrared communications port is provided in a position where infrared light can easily be exposed, such as near the display device 19, and is connected to the system controller 22 with a cable.

With an endoscope surgery system 3 relating to such a configuration, the various types of surgery data stored on the HDD 22a is storing chronologically from the beginning to the end of the surgery. In other words, the system controller 22 stores the living body data of the patient which is input from the patient monitor system 4, and the surgery-associated equipment data which is similarly input, after the surgery begins, in the HDD 22a as surgery data. At this time, the surgery data is associated to the predetermined time data such as the elapsed time from the beginning of the surgery, and is stored. Specifically, the living body data such as pulse and blood pressure is stored with the time data in the HDD 22a, and further, the surgery-associated equipment data such as the data from medical equipment such as the electrosurgical knife device 13, and the data from peripheral devices such as the receiving device 5 and the VTR 17, are stored in the HDD 22a.

As for the time data, the surgery start time can be taken as zero so as to show the elapsed time from that starting point, or time data may be data using the date and time of that day, that is to say, using the Standard Time of the time zone where the surgery is being performed.

Also, the data of the various types of events relating to the present embodiment are also stored along with the storing of the surgery data.

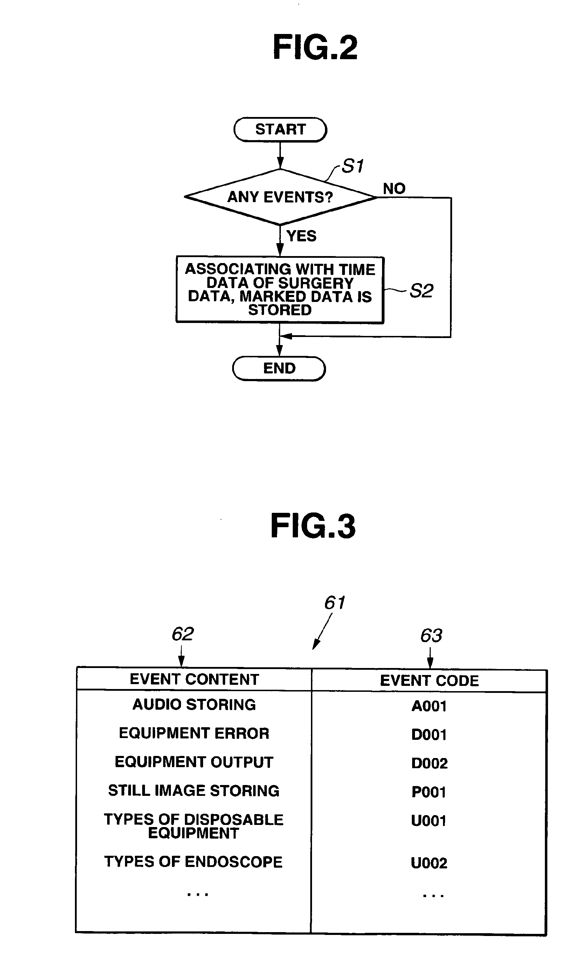

Fig. 2 is a flowchart showing event data storing processing which is executed at the same time as the surgery data storing. When the storing starts for the surgery data after surgery begins, the process of the flowchart in Fig. 2 is also executed with the system controller 22, and the event data is also stored on the HDD 22a. A central processing unit (CPU), ROM, RAM, and so forth are loaded on the system controller 22, and predetermined software programs are stored therein. Thus, by executing such programs, the system controller 22 can perform various processing to be described below. Accordingly, the system controller 22 has an operation data storing section and an event data storing section, which are realized by such programs.

First, the system controller 22 determines whether or not there is a predetermined event (step S1). This determination is made based on the data input into the system controller 22. The data input into the system controller 22 may include audio data from the microphone 33, output data from the electrosurgical knife device 13 and the like, operational data to the operational unit such as the endoscope 31, data from the RFID receiving device 5, and so forth.

In the event that Step S1 yields YES, that is to say, in the event that determination has been made that there is a predetermined event, the system controller 22 associates to the predetermined time data which is the same as the surgery data, and stores the mark data corresponding to the event in the event data table (to be described below) in the HDD 22a. Note that in the event that Step S1 yields NO, that is to say, in the event that determination has been made that there is not a predetermined event, the system controller 22 does nothing in the process in Fig. 2. Step S1 and S2 configure the event data storing section. With the present embodiment, the event data is stored in the HDD 22a wherein the surgery data is stored, but the system controller 22 can store event data in a storing device separate from the HDD 22a.

Next, the determination of whether there is an event and the storing of the mark data will be described.

First, the content or type of event is predetermined for event determination, and an event code is set for each event content. Fig. 3 is a diagram showing an example of an event code data table wherein the correlation between event content and event code is set. As shown in Fig. 3, an event code data table 61 which is an event code storing section has an event code 63 previously set, according to an event content 62.

Since the various data is input as described above, the system controller 22 performs determination as to whether a predetermined event has occurred, based on the input data. For example, the system controller 22 can determine whether or not there is an event based on the audio data input from the microphone 33, or it can determine whether or not there is an event based on the output data from the electrosurgical knife device 13 and so forth.

The case wherein determination is made as to whether or not there is an event of audio storing instructions based on audio data will be described. This is because there is the case wherein the operator desires to store the content, progress, and so forth of the surgery audibly and desires to confirm the stored content after the surgery or desires to form medical records thereof.

The system controller 22 continuously analyzes the audio data input by the microphone 33, with the audio recognition function after the surgery, and determines whether or not there is an event such as audio storing instructions. For example, audio data such as "audio storing" or "dictation" is registered in advance as instruction data for the audio storing start of the audio data, and when the audio data is recognized by the audio recognition function, the system controller 22 determines that there is an event called audio storing instructions.

In the event determination is made that there are audio storing start instructions, the system controller 22 then stores the audio input through the microphone 33 as audio data, and also stores the event data showing that there is an event of audio storing instructions into the event data table of the HDD 22a along with the time data when the audio storing instructions occurred. The time data is the same data as the time data of the surgery data, and for example, is the elapsed time from the start of the surgery to the time of the audio storing instructions.

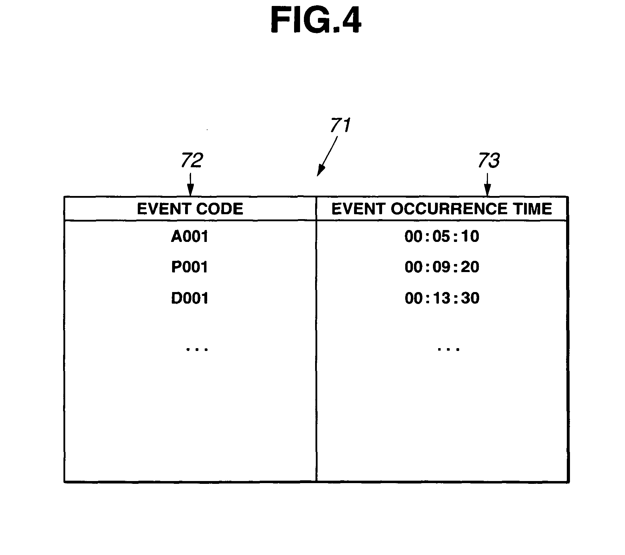

Fig. 4 is a diagram showing an example of an event data table as the event data storing section. As shown in Fig. 4, an event data table 71 includes an event code data 72 showing event content and an event occurrence time data 73.

Accordingly, when determination is made in the processing in Fig. 2 that there is an event, for example, when determination is made that there is an event called audio storing instructions, the system controller 22 stores the audio data in the predetermined storage region of the HDD 22a, and further, referencing the event code data table 61, stores the data showing the event content in the event data table 71 in the HDD 22a, for example the event code corresponding to the audio storing instructions, along with the time data of the occurrence of the event.

The audio data can be stored only for the time decided in advance, or by registering the instruction data for audio storing finishing in advance, the audio storing can be finished when the storing finishing instruction data, for example the audio data such as "audio storing finished" is recognized by the audio recognition processing.

Accordingly, in the event that determination is made that there is an event, the system controller 22 references the event code data table 61, and reads the event code of the occurred event, and stores the data of the event code and the occurrence time in the event data table 71.

Next, the case wherein determination is made as to whether or not there is an event based on output data from medical equipment will be described. This is to be effective, when the operator analyzes the surgery data after the surgery, in determining the meaning of the changes of the living body data and so forth based on whether or not there is output from medical equipment.

The system controller 22 continuously monitors the predetermined output data input from the medical equipment such as the electrosurgical knife device 13, and determines whether or not there has been an event following the start of surgery. Specifically, for example, when the operator operates the electrosurgical knife and instructs the output of the electrosurgical knife device 13, the electrosurgical knife device 13 performs output of the electrosurgical knife, and the electrosurgical knife device 13 also outputs, at the same time, data showing that output is being performed, to the system controller 22. Accordingly, by registering the electrosurgical knife output as a predetermined event in the system controller 22 beforehand, the system controller 22 can determine whether there is such an event, based on the output data from the electrosurgical knife device 13.

Also, the endoscope images during surgery are stored as still images by the operator pressing the predetermined release button provided on the operation unit of the endoscope, but such a still image stored by the release button being pressed can be called an event. This is effective when the operator desires to include the still images data in the patient chart after the surgery. Accordingly, registering pressing of a predetermined release button, i.e., instructions for still image storing of the endoscope, in advance as a predetermined event in the system controller 22, enables the system controller 22 to determine an event of data being input showing that the still image capturing button of the endoscope operating unit is pressed.

Further, error output and so forth can be recognized as events by registering in advance specified output data from medical equipment and peripheral equipment such as error output when surgery-related equipment has failed, or various types of status output of peripheral equipment, for example, the status output that a VTR tape has finished, as predetermined events in the system controller 22. This is also to be effective, when the operator analyzes the surgery data after the surgery, in determining the meaning of the changes of the living body data and so forth based on whether or not there is failure of surgery-related equipment.

Further, by registering beforehand, as an event, data input showing types of equipment such as endoscopes or treatment instruments in the system controller 22, when the operator places the solids recognition / solids identifying device, for example, an RFID tag, provided on the endoscopes and treatment instruments to be used near the RFID receiving device 5, the data showing the types of endoscopes and so forth is read, and the system controller 22 can recognize the predetermined event that there is input of equipment type data showing the types of the endoscopes and so forth. This is effective for the operator to store the equipment used during the surgery. Accordingly, the endoscopes, treatment instruments, and so forth used by the operator during surgery can be stored as event data.

Now, the determination as to whether there has been any above-described audio storing instructions can be performed without using audio recognition processing technology, by the operator operating a predetermined operating button on the remote controller 30, or by the nurses operating the operating panel 21 based on the instructions from the operator.

Further, the system controller 22 can determine that there is an event by performing a predetermined process as to the various data, and not only based on whether or not there is various data input from the surgery-related equipment. For example, the body cavity pressure data can be observed with the pneumoperitoneum device 14, and the system controller 22 can perform comparison processing of the observed pressure data with the predetermined set pressure, and if the pressure data is determined to be higher than the set pressure, determination can be made that there is an event.

Thus, the system controller 22 stores various surgery data on the HDD 22a from the start to finish of the surgery, and also when a predetermined event occurs during the surgery, stores the content of the event together with the time data on the HDD 22a.

Next, how the surgery data and event data stored thus will be used in the case of confirming surgery content during surgery after the surgery will be described.

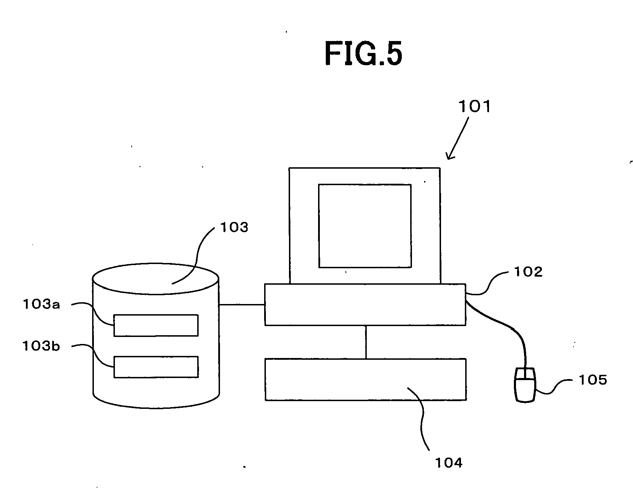

Fig. 5 is a configuration diagram showing the configuration of a surgery data display device. A surgery data display device 101 is a computer such as a personal computer. The surgery data and event data stored on the HDD 22a of the system controller 22 as described above can be stored on, with data transfer via a network, a storage device 103 which is either externally attached to or incorporated in a main unit 102 of the surgery data display device 101. Accordingly, the storage region of the storage device 103 contains a surgery data section 103a wherein surgery data is stored, and an event data section 103b wherein event data is stored. The data of the event data section 103b is the same data as that in the event data table 71 in Fig. 4. The main unit 102 is connected to a keyboard 104 which is a data input device, and a mouse 105 which is a pointing device, and further, a display device 106 is also connected, which has a screen whereupon surgery data which is the analyzed data is displayed.

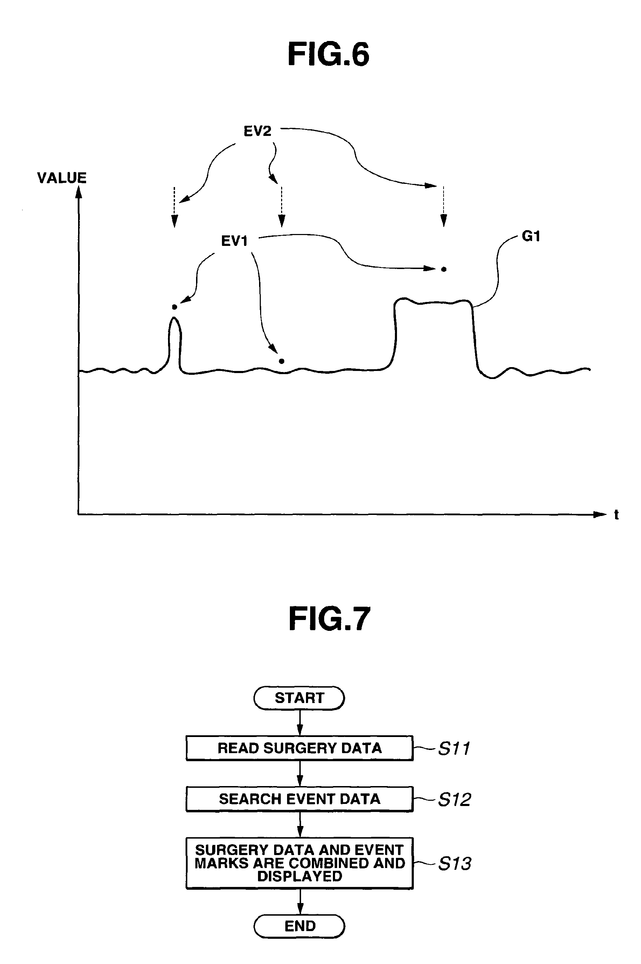

The analyst of the surgery data, for example the operator, operates the keyboard 104 or the mouse 105, and by specifying the surgery data and timeframe to be displayed on the screen, the surgery data stored on the storage device 103 can be graphed, for example, and displayed on the screen of the display device 106. Fig. 6 is a diagram showing a first display example of when one piece of surgery data is graphed and displayed. In Fig. 6, a graph G1 illustrates this graph. The changes that occur along with the course of time of the surgery data shown in the graph G1 are illustrated, and also along with the course of time of the graph G1, the occurrence or not of events are displayed near the graph G1 on the screen. In Fig. 6, only the graph G1 for one piece of surgery data is shown for simplicity of description, but if the analyst specifies multiple pieces of surgery data for the display data, then multiple graphs will be displayed along the time axis. In Fig. 6, the surgery data and the event data are displayed following the course of time of the described predetermined time, and so the analyst can observe any changes or situations occurring in the surgery data or event data following the course of time.

Also, event data is stored on the event data section 103b of the storage device 102. As described above, the event data contains event content and time data when the event occurred, as shown in Fig. 4. Accordingly, when the analyst specifies the surgery data and timeframe to be displayed on the screen, the event occurrence and occurring point in time are displayed in an easily understood manner along with the graph G1 of the surgery data. With Fig. 6, a mark (marked by a black dot in Fig. 6) EV showing that an event has occurred, or that there is event data, is displayed on the screen. The graph G1 shows this graph. The position of the mark EV1 on the screen in the horizontal direction is the position corresponding to the surgery data time along the time axis of the graph G1. With Fig. 6, the mark is shown in the position corresponding to the time wherein an event occurred, only to show that there is event data.

Fig. 7 is a flowchart showing an example of a process flow when the surgery data and event data are graphed and displayed on the screen of the display device 106.

The analyst uses the keyboard 104 or the like to command the surgery data to be graphed and displayed on the display device 106, and inputs into the surgery data display device 101. At this time, the surgery data to be graphed and displayed, and the timeframe to be graphed and displayed are included in this command. When the command is input, the processing in Fig. 7 is executed.

First, the surgery data display device 101 reads the surgery data corresponding to the command which is display instructions from the surgery data section 103a of the storage device 103 (step S11). Next, the event data having the time included in the specified timeframe of the readout surgery data is searched (S12). This search is performed by checking whether or not the various time data stored in the event occurrence time data in the event data section 103b is included within the timeframe.

The surgery data display device 101 then combines the read surgery data and the event data which is acquired by searching, and displays this as chronological data on the screen of the display device 106 (S13). This display is performed so as to combine the graph G1 of the surgery data and the predetermined mark EV1 showing the event which combines the event occurrence time with the time of the time axis on the graph G1, as shown in Fig. 6. Step S13 configures the surgery data display section and the event mark display section.

With Fig. 6, as described above, the graph G1 of the surgery data to be displayed which is specified by the analyst, and the marks EV1 which are black dots showing that there is event data, are displayed on the screen of the display device 106. With Fig. 6, the vertical axis corresponds to the values of the surgery data, and the horizontal axis corresponds to the elapsed time from the surgery start. The surgery data and the marks EV1 are displayed as chronological data following the above-described predetermined time data. Accordingly, the position for each mark EV1 to be displayed (the position in the horizontal axis direction in Fig. 6) corresponds to the time of each event occurrence, following the time axis (the horizontal axis in Fig. 6) of the surgery data.

For example, in the event that an event shows a particular output of a medical device, if the surgery data thereof changes after the occurrence of the event, the event results can confirm that the surgery data has changed. Accordingly, the analyst can view the surgery data changes corresponding to the event occurrence, and so can easily confirm the surgery content.

In Fig. 6, the position of the marks EV1 are near the graph G1, but as shown by the dotted line arrow marks EV2, these can be in a predetermined position on the screen which is farther from the graph G1.

Also, the event data section 103b also includes data showing event content, and so the marks can be set to be marks according to the event content and displayed on the graph.

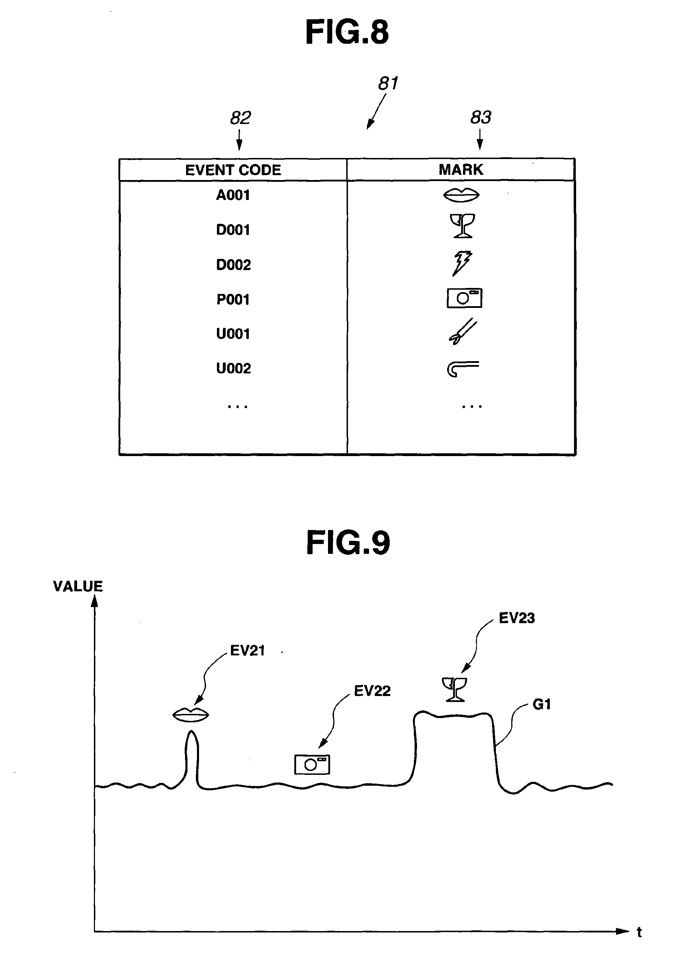

Fig. 8 is a diagram showing an example of the mark data table for setting marks in advance to correspond to event content. As shown in Fig. 8, a mark data table 81 which is the event mark data section includes an event content data 82 and a mark data 83. The mark data 83 is shown in icons or pictures in Fig. 8, but the data is code data corresponding to the pictures shown.

Accordingly, when the marks are combined with the surgery data during the processing of Step S13 in Fig. 7, the system controller 22 references the mark data table 81, reads the mark data set in advance which correspond to the events to be displayed, and based on this mark data, generates and displays the marks for the various events.

Fig. 9 is a diagram showing a second display example as to when the surgery data is graphed and displayed. The marks shown with the surgery data graph correspond to the event content, as shown in Fig. 9. In Fig. 9, a mark EV21 shows an audio storing instruction event, a mark EV22 shows a still image storing event, and a mark EV23 shows an equipment error event. The various marks are marks which correspond to the event codes 82 in the mark data table 81 shown in Fig. 8.

With the display example in Fig. 6, only the fact that there is an event is displayed, but in the display example in Fig. 9, the analyst can comprehend the event content based on the marks displayed on the display device 106.

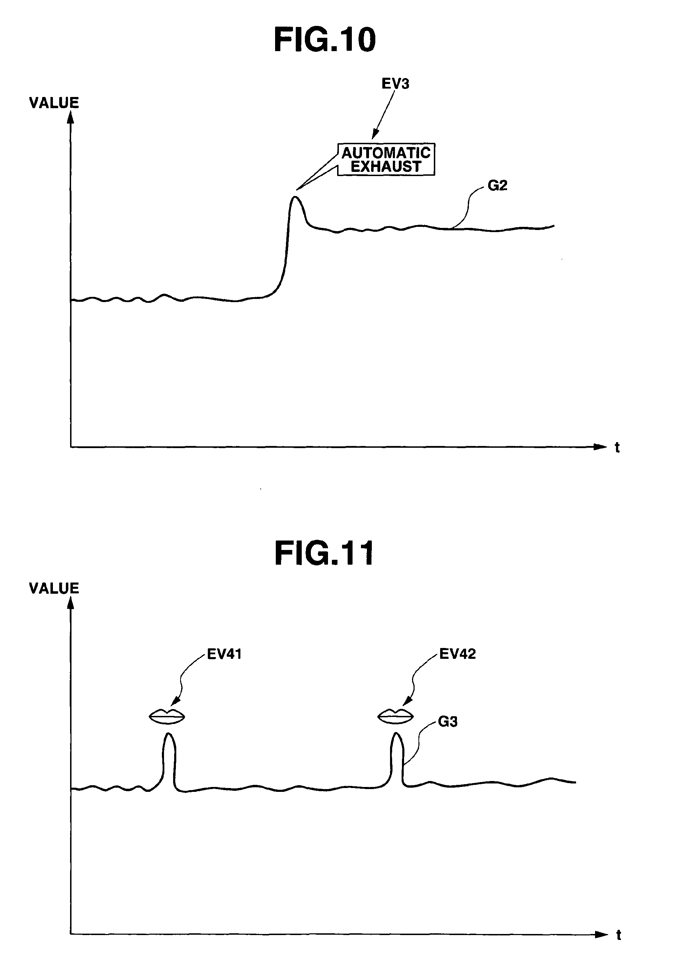

Fig. 10 is a diagram showing a third display example as to when the surgery data is graphed and displayed. As shown in Fig. 10, a mark EV3 shown with a surgery data graph G2 is displayed so as to include characters showing the event content. Fig. 10 shows a balloon display containing the characters "Automatic Exhaust".

Fig. 11 is a diagram showing a fourth display example as to when the surgery data is graphed and displayed. As shown in Fig. 11, the marks displayed with a surgery data G3 graph are only marks corresponding to audio storings. This specifies the surgery data which the analyst desires to graphically display when inputting the command for graphing and displaying the surgery data into the surgery data display device 101 using the keyboard 104 or the like, and the timeframe, and also the event to be displayed, which here is the command to extract only the audio storing instruction event. When such a command is input, the processing in Fig. 7 is executed, but only the audio storing instruction event is searched in Step S12.

As a result, the surgery data shown in Fig. 11 is displayed. In Fig. 11, the mark displayed with the surgery data graph G3 is the mark for an audio storing event. In Fig. 11, marks EV41 and EV42 illustrate audio storing events. Accordingly, after the surgery the analyst can confirm the surgery data only before and after the times at which audio storing had been performed.

Now, in the display Fig. 11, for example by moving a cursor by operating the mouse 105 and operating the button switch of the mouse 105, for example by double-clicking, on the part of the event mark EV 41 or 42, the audio data corresponding to the event mark EV41 or 42 can be played back. Also, with Fig. 11, if the event mark is a mark of an equipment error, the details of such equipment error can be displayed by double-clicking on that mark.

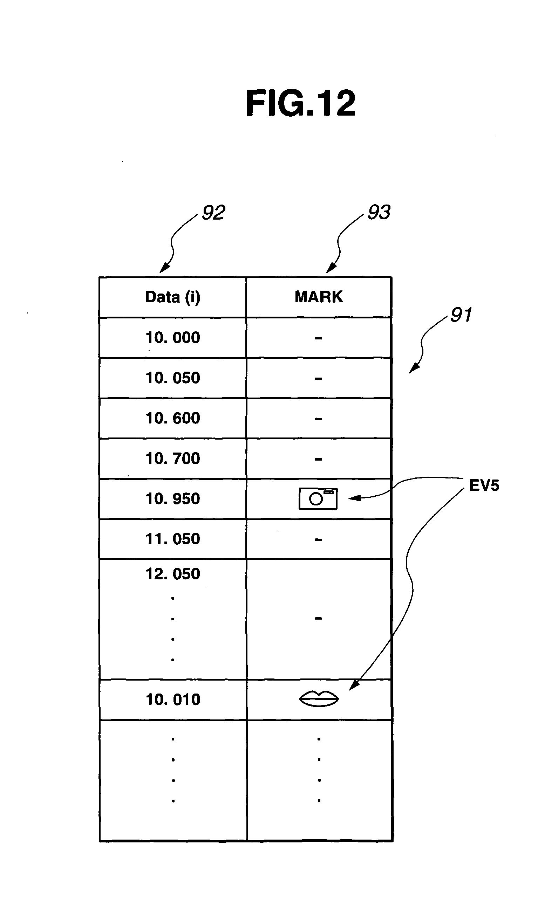

Fig. 12 is a diagram showing a fifth display example of the surgery data. As shown in Fig. 12, the surgery data and event marks EV5 are displayed in a data 91 in chart form, as chronological data. With Fig. 12, the surgery data is displayed in a surgery data column 92 in predetermined time intervals, and the event marks EV5 corresponding to the event content are displayed in a mark column 93 corresponding to the surgery data when the event occurred. If there is no event, nothing is displayed in the mark column corresponding to the surgery data, or a mark such as a dash "-" for example, is displayed to indicate that there is no event.

With Fig. 12, instead of the mark column 93, this can be an event content column, and the event content can be displayed in the event content column in text.

Also, if using a chart form such as in Fig. 12, the marks (or text) displayed in the mark column (or the event content column) 93 can be searched or sorted. Accordingly, there is the advantage wherein only specified events can be searched or continuously displayed.

Further, in the case of Fig. 12, moving a cursor by operating the mouse 105 and operating the button switch of the mouse 105 by double-clicking for example, on the part of the event marks EV5, allows the audio data corresponding to the event marks EV5 to be played back, still images can be displayed, details of the event content can be displayed, and so forth.

Thus, according to the present embodiments, the system controller correlates the various types of events to the time of the surgery data and stores these, and so the occurrence of an event, or the occurrence of an event and the content thereof, can be correlated to the surgery data with a surgery data display device. Accordingly, the surgery data and event marks can be correlated and displayed with the surgery data display device, and so the analysts such as an operator can easily confirm the surgery content.

Also, as described above, with the present invention, a mark does not only refer to a simple symbol (for example Fig. 6) showing the occurrence of an event or showing that there is event data, and is not restricted to showing the occurrence of events and so forth, but also includes symbols, reference numerals, and icons showing event content (for example Fig. 9 and Fig. 11), and further, includes icons including characters showing event content (for example Fig. 10) and text characters.

The present invention is not limited to the above-described embodiments, and can have various changes and modifications without departing from the spirit or scope of the invention.

## Claims

1. A surgery data display device, comprising:
a surgery data storing section for associating surgery data to a predetermined time and storing to a storing device;
an event data storing unit for storing event data associated with the predetermined time, according to the occurrence of a predetermined event;
a surgery data displaying section for displaying surgery data associated with the predetermined time and stored, following a course of time, on a display device; and
an event mark displaying section for displaying an event mark, which shows there is stored event data, together with the course of time, on the display device.

2. The surgery data display device according to Claim 1, wherein the event data is data showing the content of the predetermined event.

3. The surgery data display device according to either of Claims 1 or 2, wherein the predetermined event includes at least one of audio storing, surgery-related equipment output, medical equipment operation, and equipment type data input.

4. The surgery data display device according to Claim 1, wherein the event data contains data showing the content of the predetermined event, and the event mark is a mark corresponding to the content of the predetermined event.

5. A surgery data storing device, comprising:
a surgery data storing section for associating surgery data to a predetermined time and storing to a storing device; and
an event data storing section for storing event data associated with the predetermined time, according to the occurrence of a predetermined event.

6. The surgery data storing device according to Claim 5, wherein the event data is data showing the content of the predetermined event.

7. The surgery data storing device according to either of Claims 5 or 6, wherein the predetermined event includes at least one of audio storing, surgery-related equipment output, medical equipment operation, and equipment type data input.

8. A surgery data display device, comprising:
a surgery data displaying section for displaying surgery data associated with the predetermined time and stored, following the course of time, on a display device; and
an event mark displaying section for displaying an event mark, which shows there is stored event data which is associated to the predetermined time data according to the occurrence of predetermined events, together with the course of time, on the display device.

9. The surgery data display device according to Claim 8, wherein the event data contains data showing the content of the predetermined event, and the event mark is a mark corresponding to the content of the predetermined event.

10. The surgery data storing device according to Claim 9, wherein the content of the predetermined event includes at least one of audio storing, surgery-related equipment output, medical equipment operation, and equipment type data input.

11. A method of storing and displaying surgery data, the method comprising the steps of:
associating surgery data which includes at least living body data about a patient and output data from medical equipment with predetermined time data and storing the surgery data in a storing device;
associating an event data with the predetermined time data according to the occurrence of a predetermined event and storing the event data;
displaying the surgery data on a display device, following the predetermined course of time; and
displaying an event mark on the display device together with the predetermined course of time, the event mark showing there is stored event data, which is associated to the predetermined time data according to the occurrence of the predetermined event.

12. The surgery data display method according to Claim 11, wherein the event data is data showing the content of the predetermined event.

13. The surgery data display method according to either of Claims 11 or 12, wherein the predetermined event includes at least one of audio storing, surgery-related equipment output, medical equipment operation, and equipment type data input.

14. The surgery data display method according to any one of Claims 11 through 13, wherein the event mark is a mark corresponding to the content of the predetermined event.
